(19) Europäisches Patentamt
European Patent Office
Office européen des brevets

(11) Veröffentlichungsnummer: **0 071 126**
**B1**

(12) **EUROPÄISCHE PATENTSCHRIFT**

(45) Veröffentlichungstag der Patentschrift: **12.09.84**

(51) Int. Cl.³: **C 07 C 45/51**, C 07 C 47/02, C 07 C 47/28, C 07 C 47/52

(21) Anmeldenummer: **82106444.1**

(22) Anmeldetag: **17.07.82**

(54) **Verfahren zur Herstellung von Aldehyden.**

(30) Priorität: **31.07.81 DE 3130431**

(43) Veröffentlichungstag der Anmeldung: **09.02.83 Patentblatt 83/6**

(45) Bekanntmachung des Hinweises auf die Patenterteilung: **12.09.84 Patentblatt 84/37**

(84) Benannte Vertragsstaaten: **BE CH DE FR GB IT LI NL**

(56) Entgegenhaltungen:
**EP - A - 0 024 611**
**DE - A - 2 917 620**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 126 B1

**Beschreibung**

Die Erfindung betrifft ein neues Verfahren zur Herstellung von Aldehyden.

Es ist bereits bekannt, dass man Aldehyde erhält, wenn man aus entsprechenden Carbonsäurechloriden und Trialkylphosphiten gebildete α-Oxo-phosphonsäureester mit Natriumboranat zu α-Hydroxy-phosphonsäureestern reduziert und letztere mit Alkalilaugen spaltet [vgl. Chem. Ber, 103 (1970), 2984-2986, DE-OS Nr. 2934034].

Die Herstellung von Aldehyden im alkalischen Milieu ist jedoch wegen der möglichen Nebenreaktionen bzw. Folgereaktion (z.B. Phosphonat-Phosphat-Umlagerung, Aldolreaktion, Canizzaro-Reaktion) mit Nachteilen behaftet.

Es wurde nun ein Verfahren zur Herstellung von Aldehyden der Formel (I)

$$R-CHO \qquad (I)$$

in welcher R für einen gegebenenfalls substituierten Rest aus der Reihe Alkyl, Alkenyl, Alkinyl, Cycloalkyl, Cycloalkenyl, Aralkyl, Aralkenyl, Aryl oder Heteroaryl steht, gefunden, welches dadurch gekennzeichnet ist, dass man α-Hydroxy-phosphonsäureester der Formel (II)

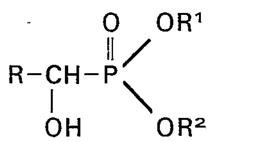

(II)

in welcher R die oben angegebene Bedeutung hat, $R^1$ und $R^2$ unabhängig voneinander für Alkyl oder Phenyl oder zusammen für Alkandiyl (Alkylen) stehen, gegebenenfalls in Gegenwart eines Verdünnungsmittels, auf Temperaturen zwischen 50 und 300°C erhitzt.

Es ist als überraschend zu bezeichnen, dass die Aldehyde der Formel (I) nach dem erfindungsgemässen Verfahren durch Pyrolyse von entsprechenden α-Hydroxy-phosphonsäureestern der Formel (II) in sehr guten Ausbeuten erhalten werden können, da eher eine thermische Phosphonat-Phosphat-Umlagerung zu erwarten war [vgl. J. Gen. Chem. USSR 38 (1968), 142-147].

Das erfindungsgemässe Verfahren weist verschiedene Vorteile gegenüber dem Stand der Technik auf. Es werden Folgereaktionen von Aldehyden, welche im alkalischen Milieu leicht eintreten (Aldolreaktion, Canizzaro-Reaktion), vermieden. Die als Koppelprodukte gebildeten Phosphorigsäureester werden nicht verseift und können durch Destillation isoliert werden. Ferner wird das Anfallen von Abwasser vermieden.

Verwendet man α-Hydroxy-benzylphosphonsäure-dimethylester als Ausgangsverbindung, so kann der Reaktionsablauf beim erfindungsgemässen Verfahren durch das folgende Formelschema wiedergegeben werden

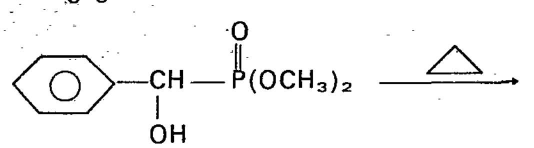

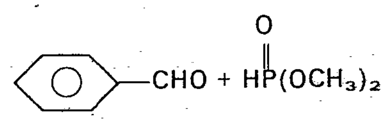

Die beim erfindungsgemässen Verfahren als Ausgangsstoffe zu verwendenden α-Hydroxy-phosphonsäureester sind durch die Formel (II) definiert. In dieser Formel stehen vorzugsweise

R für gegebenenfalls durch Fluor, Chlor, Brom, $C_1$-$C_4$-Alkoxy oder $C_1$-$C_4$-Alkylthio substituiertes $C_1$-$C_5$-Alkyl, für gegebenenfalls durch Fluor, Chlor oder Brom substituiertes $C_3$-$C_5$-Alkenyl oder $C_3$-$C_5$-Alkinyl, für gegebenenfalls durch Fluor, Chlor, Brom, $C_1$-$C_4$-Alkyl, $C_1$-$C_4$-Alkylcarbonyl, $C_1$-$C_4$-Alkoxy-carbonyl, Phenoxybenzyloxycarbonyl (welches gegebenenfalls durch Fluor, Chlor, Brom, Methyl, Methoxy und/oder Cyano substituiert ist) und/oder Cyano substituiertes $C_3$-$C_6$-Cycloalkyl, für gegebenenfalls durch Chlor, $C_1$-$C_4$-Alkyl und/oder Trifluormethyl substituiertes Benzyl oder Phenylethyl oder für gegebenenfalls durch Nitro, Fluor, Chlor, Brom, $C_1$-$C_4$-Alkyl, Trifluormethyl, $C_1$-$C_4$-Alkoxy, Trifluormethoxy, $C_1$-$C_4$-Alkylthio, Trifluormethylthio, $C_1$-$C_2$ Alkylendioxy (welches gegebenenfalls durch Fluor substituiert ist) und/oder Phenoxy (welches gegebenenfalls durch Fluor, Chlor, Brom, $C_1$-$C_4$-Alkyl, Trifluormethyl, $C_1$-$C_4$-Alkoxy, Trifluormethoxy, $C_1$-$C_4$-Alkylthio, Trifluormethylthio und/oder $C_1$-$C_4$-Alkylendioxy substituiert ist) substituiertes Phenyl. Pyridinyl oder Pyrimidinyl,

$R^1$ für $C_1$-$C_4$-Alkyl oder Phenyl, und

$R^2$ für $C_1$-$C_4$-Alkyl oder Phenyl, oder zusammen mit $R^1$ für geradkettiges oder verzweigtes $C_2$-$C_5$-Alkandiyl.

Die Ausgangsverbindungen der Formel (II) sind zum grössten Teil bekannt. Sie können nach an sich bekannten Verfahren durch Reduktion von entsprechenden α-Oxo-phosphonsäureestern hergestellt werden [vgl. Houben-Weyl, Methoden der organischen Chemie, 4. Auflage, Band 12/1, S. 512, Thieme-Verlag, Stuttgart 1963; Israel J. Chem. 4 (1966), 255; *ibid.* 9 (1971), 33; DE-OS Nrn. 2917620 und 2934034.

Als Beispiele für die Ausgangsverbindungen der Formel (II) seien genannt:
α-Hydroxy-ethyl-, α-Hydroxy-n-propyl-, α-Hydroxy-n-butyl-, α-Hydroxy-iso-butyl-, α-Hydroxy-n-pentyl-, α-Hydroxy-iso-pentyl-, α-Hydroxy-neopentyl-, (α-Hydroxy-β, β-dimethyl-propyl-), α-Hydroxybenzyl-, α-Hydroxy-3-methyl-benzyl-, α-Hydroxy-4-methyl-benzyl-, α-Hydroxy-3,4-dimethyl-benzyl-, α-Hydroxy-4-ethyl-benzyl-, α-Hydroxy-4-iso-propyl-benzyl-, α-Hydroxy-4-tert.-butyl-benzyl-, α-Hydroxy-4-fluor-benzyl-, α-Hydroxy-4-chlor-benzyl-, α-Hydroxy-2,4-dichlor-benzyl-, α-Hydroxy-4-brom-benzyl-, α-Hydroxy-3-brom-4-fluor-benzyl-, α-Hydroxy-4-methoxy-benzyl-, α-Hydroxy-3,4-dimethoxy-benzyl-, α-Hydroxy-2-trifluormethyl-benzyl-, α-Hydroxy-3-trifluormethyl-benzyl-, α-Hydroxy-4-trifluormethyl-benzyl-, α-Hydroxy-3-phenoxy-benzyl-, α-Hydroxy-4-fluor-3-phenoxy-benzyl- und α-

Hydroxy-4-trifluormethoxy-benzyl-phosphonsäure-dimethylester und -diethylester; ferner α-Hydroxy-α-(2,2-dichlor-1-methyl-cyclopropyl)-methanphosphonsäure-dimethylester und -diethylester, α-Hydroxy-α-(3-methoxycarbonyl-2,2-dimethyl-cyclopropyl)-methanphosphonsäure-dimethylester und -diethylester, α-Hydroxy-α-(3-ethoxycarbonyl-2,2-dimethyl-cyclopropyl)-methanphosphonsäure-dimethylester und-diethylester sowie α-Hydroxy-α-(3-cyano-2,2-dimethyl-cyclopropyl)-methanphosphonsäure-dimethylester und -diethylester.

Das erfindungsgemässe Verfahren wird ohne oder in Gegenwart eines Verdünnungsmittels durchgeführt. Als solche kommen vorzugsweise inerte organische Lösungsmittel in Betracht. Hierzu gehören insbesondere gegebenenfalls halogenierte Kohlenwasserstoffe, wie Toluol, Xylol, Chlorbenzol, Dichlorbenzol, Dekalin, sowie Ether, wie Glycoldimethylether und Diglycoldimethylether.

Die Reaktionstemperaturen können bei der Durchführung des erfindungsgemässen Verfahrens in einem grösseren Bereich variiert werden. Im allgemeinen arbeitet man bei Temperaturen zwischen 50 und 300°C, vorzugsweise zwischen 100 und 250°C, insbesondere zwischen 150 und 220°C.

Der Druck kann bei Durchführung des erfindungsgemässe Verfahrens ebenfalls in einem grösseren Bereich variiert werden. Im allgemeinen arbeitet man im Bereich zwischen 0,0001 und 2000 mbar, vorzugsweise zwischen 0,01 und 1500 mbar.

Das erfindungsgemässe Verfahren kann nach üblichen Pyrolysemethoden durchgeführt werden. In einer bevorzugten Ausführungsform wird das Verfahren in einer gegebenenfalls auf die erforderliche Reaktionstemperatur vorgeheizten Destillationsapparatur durchgeführt, in welche die Ausgangsverbindung der Formel (II), gegebenenfalls in einem Verdünnungsmittel gelöst, eindosiert wird, während synchron das Produkt der Formel (I) und gegebenenfalls der als Koppelprodukt gebildete Phosphorigsäureester und das Verdünnungsmittel gegebenenfalls unter vermindertem Druck abdestilliert wird. Man kann auch den Ausgangsstoff der Formel (II) komplett in der Apparatur vorlegen und allmählich auf die erforderliche Reaktionstemperatur bringen, wobei die Produkte ebenfalls durch Destillation isoliert werden.

Wesentlich bei der Durchführung des Verfahrens ist, den gebildeten Aldehyd aus dem Reaktionsgemisch möglichst rasch zu entfernen.

Die Produkte können bei Bedarf durch eine weitere (fraktionierte) Destillation gereinigt werden.

Die erfindungsgemäss herzustellenden Verbindungen können zum Teil beispielsweise als Zwischenprodukte für Schädlingsbekämpfungsmittel verwendet werden (vgl. DE-OS Nr. 2934034).

*Herstellungsbeispiele*

*Beispiel 1*

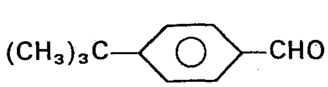

27,2 g (0,01 mol) α-Hydroxy-4-tert.-butyl-benzyl-phosphonsäure-dimethylester werden in 25 ml Toluol gelöst und aus einem Tropftrichter in eine auf 180°C vorgeheizte Destillationsapparatur mit kleiner Vigreux-Kolonne, an die Wasserstrahlvakuum angelegt ist, getropft. Das Destillat wird im Hochvakuum fraktioniert destilliert. Man erhält 13,8 g (81,5% der Theorie) 4-tert.-Butyl-benzaldehyd vom Siedepunkt 72°C/0,15 mbar.

*Beispiel 2*

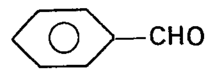

122 g (0,5 mol) α-Hydroxy-benzyl-phosphonsäure-diethylester werden in einer Destillationsapparatur im Wasserstrahlvakuum auf 165-190°C/12 mbar erhitzt. Es destillieren 78 g eines Destillates über, das laut GC zu 58% aus Benzaldehyd und zu 38,5% aus Diethylphosphit besteht. Das entspricht einer Ausbeute an Benzaldehyd von 85,4% der Theorie. Die Ausbeute kann auf über 95% gesteigert werden, wenn mit Hilfe eines Dünnschichtverdampfers gearbeitet wird.

*Beispiel 3*

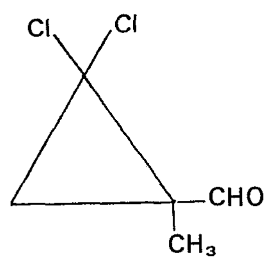

20 g (0,0687 mol) α-Hydroxy-methyl-(2,2-dichlor-1-methylcyclopropyl)-phosphonsäure-dimethylester werden im Wasserstrahlvakuum destilliert. Bei 170-180°C/12 mbar destillieren 17,4 g Produkt über, das zu 49,5% aus Dimethylphosphit und zu 43,6% aus 2,2-Dichlor-1-methyl-1-formyl-cyclopropan besteht. Durch fraktionierte Destillation wird der Aldehyd rein gewonnen. Siedepunkt 68°C/18 mbar.

*Beispiel 4*

$$(CH_3)_3C - CHO$$

31 g (0,138 mol) α-Hydroxy-neo-pentyl-phosphonsäure-diethylester werden bei Normaldruck in einer Destillationsapparatur auf 180-190°C erhitzt. Das Destillat (65-80°C) (10,2 g) besteht zu 97,6% aus Dimethylpropionaldehyd. Ausbeute: 83,4% der Theorie.

*Beispiel 5*

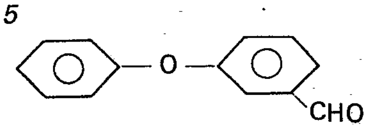

59 g 3-Phenoxy-α-hydroxy-benzyl-phosphonsäure-diethylester werden in 150 ml Toluol gelöst und in eine auf 220°C vorgeheizte Destillationsapparatur mit angelegtem Wasserstrahlvakuum getropft. Es destilliert ein Gemisch aus Toluol, Diethylphosphit und 3-Phenoxybenzaldehyd über. Durch fraktionierte Destillation erhält man 33,2 g

(83,8% der Theorie) reinen 3-Phenoxybenzaldehyd vom Siedepunkt 138-145°C/0,1 mbar.

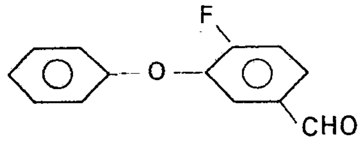

3-Phenoxy-4-fluor-benzaldehyd wird analog vorstehendem Beispiel in 85%iger Ausbeute erhalten.

*Beispiel 6*

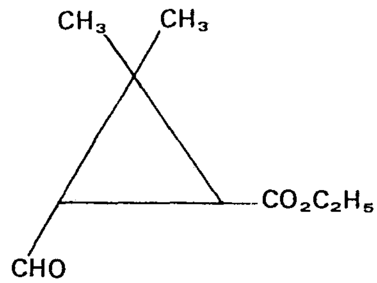

30,8 g (0,1 mol) 2,2-Dimethyl-3-(hydroxy-diethylphosphonyl)-methyl-cyclopropan-1-carbonsäure-ethylester werden in einer Destillationsapparatur auf 180-200°C erhitzt. Bei dieser Temperatur und 12 mbar Druck destillieren 28,7 g Produkt über, die nochmals redestilliert werden. Man erhält 14,8 g (87% der Theorie) Caronaldehyd vom Siedepunkt 61-65°C/0,1 mbar.

Analog den vorausgehend beschriebenen Beispielen konnten die folgenden Verbindungen erhalten werden

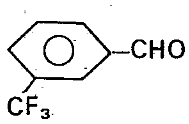
Ausbeute 87%
Siedepunkt: 72-74°C/15 mbar

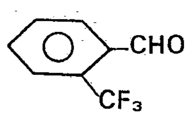
Ausbeute 81%
Siedepunkt: 62-65°C/15 mbar

*Herstellung der Ausgangsstoffe*

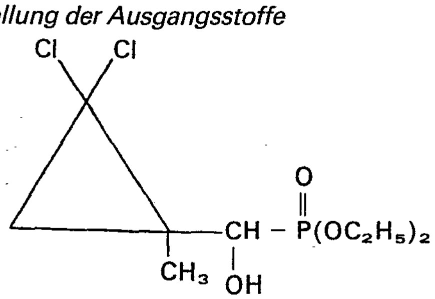

28,9 g (0,1 mol) 2,2-Dichlor-1-methyl-cyclopropyl-carbonylphosphonsäure-diethylester und 1 g Tetrabutylammoniumbromid werden in 100 ml Toluol vorgelegt und 1,134 g (0,03 mol) Natriumboranat in 20 ml Wasser bei 15-20°C zugetropft. Nach einstündigem Nachrühren wird neutral gestellt, die Toluolphase abgetrennt, mit Wasser gewaschen, getrocknet und eingeengt. Man erhält 24,4 g eines farblosen Öls, das nach einiger Zeit erstarrt. Schmelzpunkt 57°C.

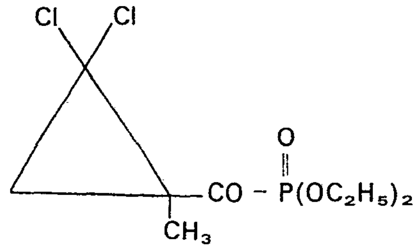

Zu 61 g (0,368 mol) Triethylphosphit werden bei Raumtemperatur 69 g (0,368 mol), 2,2-Dichlor-1-methyl-cyclopropan-1-carbonsäurechlorid getropft. Man rührt noch 1 h nach und destilliert im Hochvakuum bei 50°C die leicht flüchtigen Bestandteile ab. Der Rückstand besteht aus 127 g 2,2-Dichlor-1-methyl-cyclopropyl-carbonylphosphonsäure-diethylester (farbloses Öl).

## Patentanspruch

Verfahren zur Herstellung von Aldehyden der Formel I

R−CHO (I)

in welcher R für einen gegebenenfalls substituierten Rest aus der Reihe Alkyl, Alkenyl, Alkinyl, Cycloalkyl, Cycloalkenyl, Aralkyl, Aralkenyl, Aryl oder Heteroaryl steht, dadurch gekennzeichnet, dass man α-Hydroxy-phosphonsäureester der Formel II

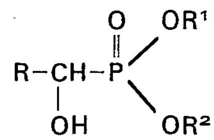 (II)

in welcher R die oben angegebene Bedeutung hat, $R^1$ und $R^2$ unabhängig voneinander für Alkyl oder Phenyl oder zusammen für Alkandiyl (Alkylen) stehen, gegebenenfalls in Gegenwart eines Verdünnungsmittels, auf Temperaturen zwischen 50 und 300°C erhitzt.

## Revendication

Procédé pour la fabrication d'aldéhydes de formule I

R−CHO (I)

dans laquelle R représente un reste éventuellement substitué de la série alkyle, alcényle, alcynyle, cycloalkyle, cycloalcényle, arylalkyle, arylalcényle, aryle ou hétéroaryle, caractérisé en ce que l'on chauffe des esters d'acides α-hydroxy-phosphoniques de formule II

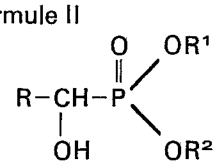 (II)

dans laquelle R a la signification indiquée ci-dessus, $R^1$ et $R^2$ représentent, indépendamment l'un de l'autre, un reste alkyle ou phényle ou, ensemble, un reste alkylène, à des températures entre 50 et 300°C, éventuellement en présence d'un agent diluant.

## Claim

Process for the preparation of aldehydes of the formula I

$$R-CHO \qquad (I)$$

in which R represents an optionally substituted radical from the series comprising alkyl, alkenyl, alkinyl, cycloalkyl, cycloalkenyl, aralkyl, aralkenyl, aryl or heteroaryl, characterised in that α-hydroxyphosphonic acid esters of the formula II

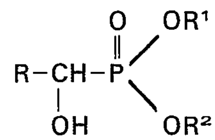

(II)

in which R has the meaning given above and $R^1$ and $R^2$ independently of one another represent alkyl or phenyl or together represent alkanediyl (alkylene), are heated to temperatures between 50 and 300°C, if appropriate in the presence of a diluent.